# EUROPEAN PATENT SPECIFICATION

(11) **EP 0 282 301 B1**
(45) Date of publication and mention of the grant of the patent: **22.12.1993**
(21) Application number: 88302088.5
(22) Date of filing: 10.03.1988
(51) Int. Cl.: A61L 9/12, A61L 9/015

(54) **Deodorizing apparatus**
Desodoriervorrichtung
Désodorisateur

(30) Priority: 13.03.1987 JP 37520/87 U; 25.03.1987 JP 44989/87 U; 25.03.1987 JP 44990/87 U; 17.04.1987 JP 59013/87 U; 20.04.1987 JP 60501/87 U; 21.04.1987 JP 60369/87 U; 22.04.1987 JP 60892/87 U; 24.04.1987 JP 62918/87 U; 17.07.1987 JP 110211/87 U
(43) Date of publication of application: 14.09.1988
(73) Proprietor: SHARP KABUSHIKI KAISHA, Osaka 545 (JP)
(72) Inventor: Miyakami, Jungi, Suita-shi, Osaka, (JP); Yoshioka, Yuzo, Osaka, (JP); Otoji, Kazuhiko, Osaka, (JP); Nakagawa, Hiroyasu, Osaka, (JP); Shirouzu, Toshiyuki, Yao-shi, Osaka (JP); Yada, Fumio, Yao-shi, Osaka, (JP)
(74) Representative: Brown, Kenneth Richard

(56) References cited:
- EP-A- 0 057 624
- DE-A- 3 639 396
- FR-A- 2 581 565

## Description

The present invention relates to a deodorizing apparatus for use in storage chambers such as refrigerators for removing odours from air by converting the oxygen in the air to ozone.

Conventionally, for example, active carbon is used in refrigerators for removing the odours released from the foodstuffs stored therein, utilizing the adsorbing property of the carbon. However, active carbon needs replacement once it is saturated with adsorbed odours. Active carbon has the further drawback of necessitating replacement at short intervals of time, since it is difficult to recognise whether saturation has been reached or not.

To obviate these drawbacks, deodorizing apparatus have already been proposed in which some of the oxygen in odour-containing air is converted by silent discharge to ozone. The ozone then reacts with air on the surface of a catalyst provided to facilitate deodorization. With these deodorizing apparatus, the components of odours are decomposed on oxidation by the deodorizing catalyst activated with ozone, and at the same time, an excess of ozone is decomposed to oxygen (20₃ → 30₂), which is discharged from the apparatus. (See, for example, Examined Japanese Utility Model Publication SHO 60-24351).

However, the convention deodorizing apparatus has the drawback that when the electric motor thereof stops its operation due to a malfunction or for some other cause, the remaining ozone flows out of the case through an air inlet. This has an adverse effect on the interior material of the storage chamber and on the food stored therein.

The apparatus has another drawback. The deodorising catalyst, when used for a prolonged period of time, exhibits a decreased ozone decomposition efficiency. Thus, there is an increase in the amount of residual ozone in the oxygen flowing out of the apparatus, which gives a higher residual ozone concentration in the interior of the storage chamber which adversely affects the interior material and the food stored therein.

A first aspect of the present invention provides a deodorizing apparatus comprising means for generating an airflow through a case from an inlet to an outlet of the case, means for introducing ozone into the airflow at a position between said inlet and outlet and first means disposed in said airflow downstream of said position for accelerating the decomposition of odours and ozone in the airflow, characterised by the provision of a second means for accelerating the decomposition of odours and ozone located upstream of said position.

Thus, the invention provides a deodorizing apparatus of the type described above which is characterised in that the residual ozone is in no way allowed to flow out of the case of the apparatus even if the electric fan stops its operation.

A second aspect of the present invention provides a deodorizing apparatus comprising means for generating an airflow through a case from an inlet to an outlet of the case, means for introducing ozone into the airflow at a position between said inlet and outlet and means disposed in said airflow downstream of said position and for accelerating the decomposition of odours and ozone in the airflow, characterised in that said outlet is above said inlet, and a motor of said airflow generating means is located in the case upstream of said position.

In a preferred embodiment, the ozone generator is operated intermittently at a specified interval so as to produce a predetermined amount of ozone in accordance with the capacity and construction of the storage chamber in which the deodorizing apparatus is installed. Additionally, the ozone concentration within the storage chamber is made readily controllable to not higher than a given regulation value, utilizing such variation in the interior ozone concentration that it increases owing to the presence of residual ozone while the generator is in operation but decreases by virtue of the spontaneous decomposition of ozone to oxygen (20₃ → 30₂), leakage, etc, while the generator is out of operation.

Specific embodiments of the invention will now be described with reference to the drawings in which:
Fig. 1 is a perspective view showing the basic construction of a deodorizing apparatus embodying the invention;
Fig. 2 is a perspective view showing the basic construction of another deodorizing apparatus embodying the invention;
Fig. 3 is a perspective view showing a deodorizing apparatus in the form of a unit and having the basic construction of Fig. 1;
Fig. 4 is a fragmentary section view of Fig. 3;
Fig. 5 is a diagram showing the electric circuit of the apparatus of Fig. 3;
Fig. 6 is a view showing the interior arrangement of a refrigerator having incorporated therein the apparatus of Fig. 1;
Fig. 7 is a view showing the interior arrangement of a refrigerator having incorporated therein the apparatus of Fig. 2;
Fig. 8 is a block diagram showing a control circuit for the apparatus of Fig. 6 and 7;
Figs. 9 and 10 are time charts for illustrating the operation of the control circuit of Fig. 8;
Figs. 11 and 12 are fragmentary diagrams showing the circuit of Fig. 8 in greater detail;
Fig. 13 is a graph showing the relation between the amount of ozone produced and the primary voltage of a high voltage generating unit as established by the deodorizing apparatus of the invention and the conventional apparatus for comparison; and
Fig. 14(a) to Fig. 14(c) are flow charts illustrating the operation of the control circuit of Fig. 8.

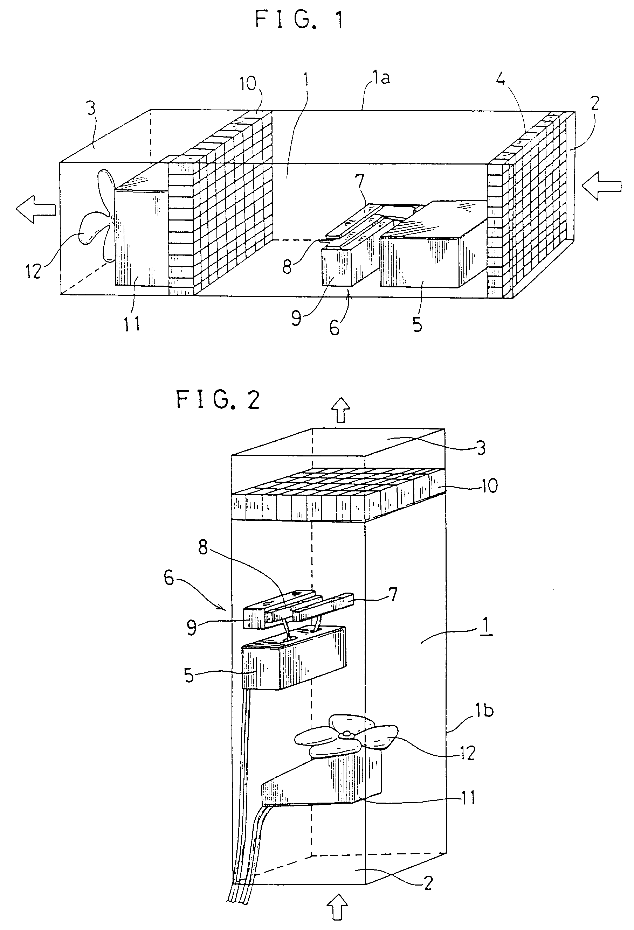

Fig. 1 shows the basic construction of a deodorizing apparatus embodying the invention.

The apparatus of Fig. 1 has a case 1a providing an airflow channel 1 and having at its front and rear ends an air inlet 2 for taking in air containing odours and an air outlet 3. The upstream end of the airflow channel 1, where the inlet 2 is positioned for the air to start flowing through the channel, is provided with a deodorizing catalyst 4 having a honeycomb structure for accelerating the decomposition of the odours and ozone. A high voltage generating unit 5 is disposed in the channel 1 downstream from the catalyst 4. Disposed in the channel downstream from the unit 5 is an ozone generator 6 which comprises a high-voltage electrode 7, a low-voltage electrode 8 and an electrode support 9. The airflow channel 1 is further provided with a deodorizing catalyst 10, similar to catalyst 4 and positioned downstream from the ozone generator 6, a motor 11 and a fan 12 attached to the motor 11.

The deodorizing catalysts 4, 10 have catalytic activity to adsorb or decompose odour components and also to decompose an excess of ozone. More specifically, these catalysts comprise a composite substrate of titania with silica and manganese oxide coating the substrate surface.

When the deodorizing apparatus of the above construction is driven, the motor 11 rotates the fan 12, whereby odour-containing air is taken into the apparatus through the air inlet 2. At the same time a high voltage is supplied from the high voltage generating unit 5 to the ozone generator 6, giving rise to a silent discharge across the high-voltage electrode 7 and the low-voltage electrode 8. While the air introduced into the channel 1 is sent toward the air outlet 3 by the fan 12, a portion of the air has its oxygen converted to ozone (0₃) by the silent discharge. The air flowing into the case 1a contains odour components and passes through the deodorizing catalyst 10 along with the ozone produced by the generator 6. The catalyst 10, activated with the ozone, decomposes the odour components on oxidation, and at the same time, decomposes an excess of ozone to oxygen (20₃ → 30₂), which then exits from the case 1a through the outlet 3.

If only the fan 12 is out of operation owing to a malfunction or for some other cause and the ozone generator 6 is in normal operation, the air does not always flow from the inlet 2 toward the outlet 3 (forward flow) but is likely to flow from the outlet 3 toward the inlet 2 (reverse flow). However, according to the first aspect of the present invention, if a reverse flow occurs while ozone is being produced in the apparatus, the ozone within the apparatus is decomposed to oxygen by the deodorizing catalyst 4 before passing through the inlet 2. This eliminates the likelihood of the remaining ozone flowing out from the inlet 2, rendering the apparatus usable with improved safety.

Fig. 2 is a perspective view showing the basic construction of another embodiment of the invention.

The deodorising apparatus of Fig. 2 has an upright case 1b providing an airflow channel 1 and has at its lower and upper ends an air inlet 2 for taking in odour-containing air and an air outlet 3, respectively. Between the inlet 2 and the outlet 3, the channel 1 is provided with a motor 11, fan 12 attached to the motor 11, ozone generator 6 and honeycomb deodorizing catalyst 10 which are arranged downstream from the inlet 2 toward the outlet 3 in the order mentioned.

When the apparatus is driven, the motor 11 is driven to rotate the fan 12, whereby odour-containing air is taken into the flow channel 1, while a silent discharge occurs in the ozone generator 6 across a high voltage electrode 7 and a low-voltage electrode 8 to produce ozone.

The air taken into the channel 1 is sent toward the outlet 3 by the fan. At this time, the odour components in the air come into contact with the deodorizing catalyst 10 which is activated by the ozone and are thereby subjected to accelerated oxidation to decompose into odourless components. An excess of ozone is also decomposed to oxygen when passing through the catalyst 10.

While the fan 12 is in normal operation, the ozone from the generator 6 is decomposed by passing through the catalyst 10 as stated above and therefore does not flow out of the channel 1. Nevertheless, if the motor stops for one cause or another, in the case of the conventional apparatus, the ozone would reversely flow toward the air inlet and flow out therefrom; whereas the reverse flow is avoidable using the present deodorizing apparatus of the upright type wherein the air inlet 2, motor 11, ozone generator 6, deodorizing catalyst 10 and air outlet 3 are arranged from the lowermost position upward. Even if the motor 11 of the present apparatus becomes locked and stops the fan 12, the heat emanating from the locked motor 11 produces an upward air current, whereby the ozone produced by the generator 6 is forced upward and precluded from flowing out via the inlet 2.

Deodorizing apparatus having the basic constructions of Figs. 1 and 2 are divided generally into those of the unit type which can be removably installed in storage chambers such as refrigerators, and those which are incorporated into storage chambers.

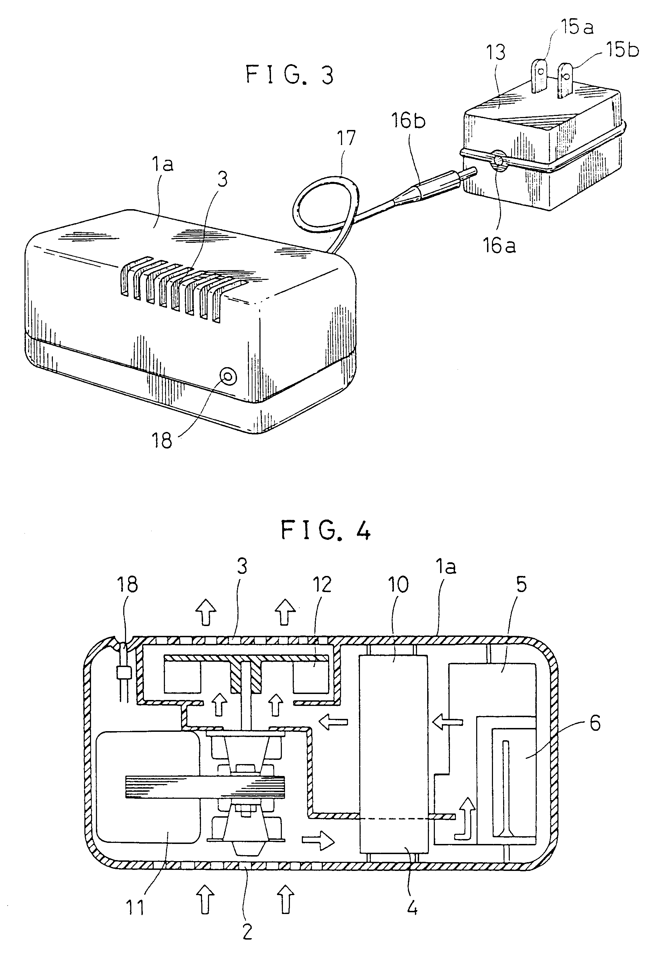

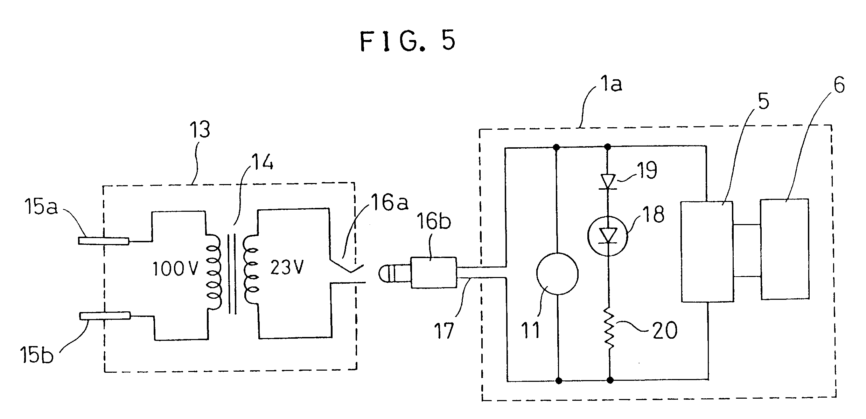

Fig. 3 is a perspective view of a deodorizing apparatus of the unit type having the basic construction of Fig. 1, Fig. 4 is a view partly in section and showing the same, and Fig. 5 is a diagram showing the electric circuit of the apparatus of Fig. 3. These drawings show a transformer case 13 housing a transformer 14 for stepping down a.c. 100 V to a.c. 23 V, blades 15a, 15b connected to the primary winding of the transformer 14 and to be inserted into an a.c. 100 V receptacle, a jack 16a for removably receiving therein a plug 16a, a two-core cable 17 having the plug 16b at its one end and fixed at the other end to the case 1a, an LED 18, a diode 19 for rectifying the alternating current supplied through the cable 17, and a resistor 20 for setting a current for the LED 18. Throughout Figs. 1 and Figs. 3 to 5, like parts are designated by like reference numerals.

When the apparatus is in use, the case 1a is placed on a shelf within an unillustrated refrigerator having a door, the forward end of the cable 17 is withdrawn from the refrigerator through a clearance between the refrigerator body and its door, and the cable 17 is fixed to the inner wall of the refrigerator with an adhesive tape. Alternatively, the blades 15a, 15b are inserted into an a.c. 100 V receptacle on the interior wall of the room to fix the case 13. The plug 16b at the cable end is inserted into the jack 16a, thereby turning on the LED 18 and driving the motor 11 and the ozone generator 6. The odour-containing air within the refrigerator is taken into the case 1a, deodorized and discharged therefrom as indicated by arrows in Fig. 4. The transformer 14 serves to step down the voltage to be supplied by the two-core cable 17 and to permit the use of the cable 17 of reduced size which can be drawn out from the refrigerator.

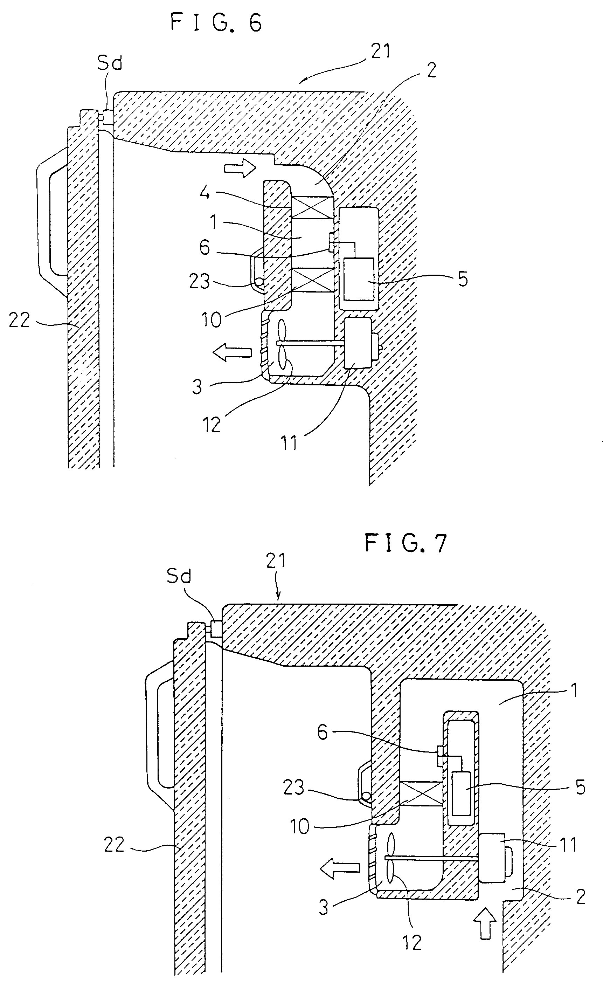

Fig. 6 is a view illustrating the interior arrangement of a refrigerator having incorporated therein the apparatus of Fig. 1, and Fig. 7 is a view showing the apparatus of Fig. 2 is similarly incorporated into a refrigerator. With reference to Figs. 6 and 7, indicated at 21 is the refrigerator, at 22 the door thereof, at Sd a door switch which is operable with the closing of the door, and at 23 a temperature sensor for detecting the internal temperature of the refrigerator. Throughout Figs. 1, 2, 6 and 7, like parts are designated by like reference numerals or symbols. With reference to Fig. 7, the airflow channel 1 is U-shaped, and the fan 12, which is disposed at the air outlet 3, is driven by the motor 11 which is positioned at the air inlet 2.

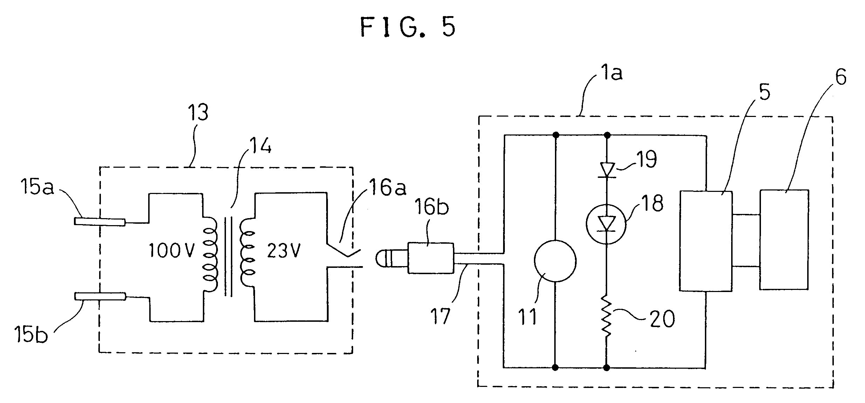

Fig. 8 is a fragmentary block diagram showing a control circuit for controlling the deodorizing apparatus of Fig. 6, as well as of Fig. 7. The control circuit includes a microcomputer 24 comprising a ROM, RAM, CPU and I/O ports, a 100 V a.c. power supply 25, switching elements Sf, So, such as triacs or rely contacts, a current detecting circuit 26 for detecting the presence or absence of the current for driving the motor 11, and a constant-voltage circuit 27. The power supply 25 supplies power to the motor 11 via the switching element Sf and the current detecting circuit 26 and also to the high voltage generating unit 5 by way of the switching elements Sf, So and the constant-voltage circuit 27. The microcomputer 24 receives signals from the door switch Sd, the temperature sensor 23 and the current detecting circuit 26 to control the on-off operation of the switching elements Sf, So.

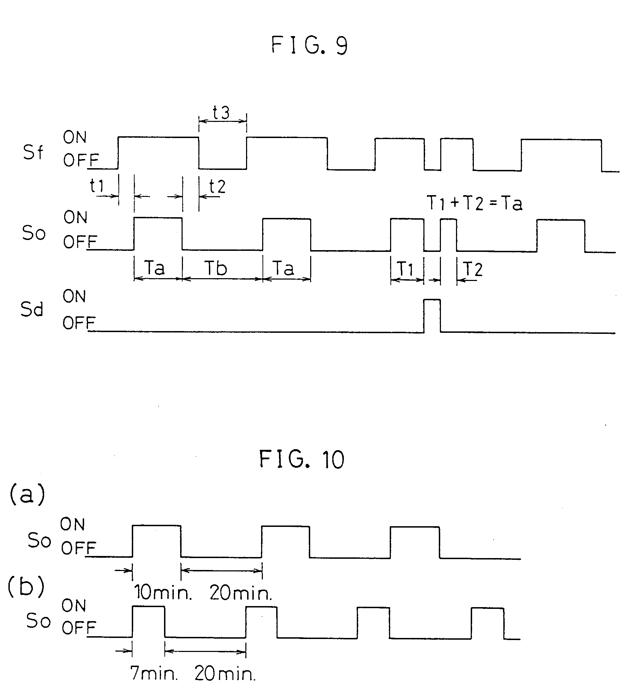

Fig. 9 is a time chart showing the operations of the switching elements Sf, So and the door switch Sd, and Fig. 10 is a time chart showing a change in the on-period of the switching element So controlled according to the output of the temperature sensor 23.

With reference to these drawings, the operations of the deodorizing apparatus and the feature of the operation will be described generally.

The microcomputer 24 includes timers (Ta timer and Tb timer), which function to hold the switching element So on during a period of time Ta, maintaining the ozone generator 6 in operation during this period (Ta mode), and to subsequently hold the switching element So off during a time interval Tb, maintaining the ozone generator 6 out of operation during this interval (Tb mode) as shown in Fig. 9. This cycle is repeated. According to the present embodiment, Ta is set to 10 minutes, and Tb to 20 minutes, in view of the capacity of the refrigerator 21 and the ability of the deodorizing catalyst 10. Thus, the ozone generator 6 is driven intermittently for 10 minutes at intervals of 20 minutes.

As already stated, the ozone decomposition efficiency of the deodorizing catalyst 10 is not always 100% but gradually decreases while in use for a long period of time, permitting the residual ozone to flow out from the air outlet 3 into the refrigerator, with the result that the ozone concentration in the refrigerator exceeds a given regulation value in several tens of years. According to the invention, however, the ozone generator 6 is driven intermittently at the above-mentioned interval so as to maintain the interior ozone concentration below the regulation value at all times. Consequently, the interior ozone concentration will not exceed the regulation value of 0.1 ppm for 20 years, rendering the deodorizing apparatus operation with exceedingly high safety.

The microcomputer 24 further has incorporated therein a long-period timer (3-day timer for measuring the period of 3 days in the present embodiment), which functions to forcibly turn off the ozone generator 6 and the motor 11 if the door 22 is held closed for more than a specified period of time, i.e. for more than 3 days, to prevent an increase in the interior ozone concentration. Further, in response to a closing signal (Sd=OFF) from the door switch Sd, the microcomputer 24 energises the motor 11 as will be described later and drives the ozone generator 6 for time periods Ta at intervals Tb as already stated above. If the door is opened during this mode of control, the motor 11 and the ozone generator 6 are deenergised with the door movement. The motor 11 and the generator 6 are thereafter energised with the closing of the door, whereupon the Ta or Tb timer resumes its time measuring operation to hold the ozone generator 6 in operation (or out of operation) during the remaining period of time.

Accordingly, the opening or closing of the door produces no variations in the on-period or off-interval of the apparatus.

Further, if the door is held closed for a long period of time, e.g., for days, the ozone generator 6 and the motor 11 are forcibly deenergized to prevent the interior ozone concentration from increasing.

The ozone generator has the problem that the silent discharge is affected by the temperature and humidity around the discharge electrodes, hence there are variations in the amount of ozone produced. Additionally, odour are perceived strongly at high temperatures but less strongly at low temperatures, so that there is a need to control the deodorizing apparatus according to the temperature.

The present embodiment is therefore adapted to control the amount of ozone to be produced according to the internal temperature of the refrigerator detected by the temperature sensor 23. More specifically, the apparatus is driven intermittently with a cycle wherein the switching element So is on (for the production of ozone) for 10 minutes and off for 20 minutes as shown in Fig. 10(a) while the internal temperature Th is not lower than a set temperature Ts (5°C); whereas if the temperature Th becomes lower than the set temperature Ts, the cycle is changed so that the switching element So is on for 7 minutes and off for 20 minutes as seen in Fig. 10(b) in order to decrease the amount of ozone to be produced. Moreover, a plurality of temperature settings may be provided to give a more minutely altered time ratio for the intermittent operation to control the amount of ozone relative to the internal temperature Th. If the intermittent operation time ratio is adjusted similarly using a humidity sensor instead of the temperature sensor 23, the decrease in the amount of ozone due to an increase in humidity can be compensated for.

Next, a description will be given of the operation of the motor 11 for driving the fan 12. As already described, the motor 11 is controlled by the switching element Sf, which in turn is turned on and off by signals from the microcomputer 24. As shown in Fig. 9, the ON signal for the switching element Sf is given earlier than the ON signal for the switching element So for operating the high voltage generating unit 5 by a period of time tl. The OFF signal for the switching element Sf is produced a period time t2 after an OFF signal is given to the switching element So.

Thus, when the high voltage generating unit 5 is to be operated, the motor 11 is first operated, and the unit 5 is initiated into operation the period of time tl thereafter. Further, upon lapse of the specified time t2 after the unit 5 is brought out of operation, the motor 11 is deenergized. This mode of control causes the residual ozone to flow in the forward direction, preventing the ozone from reversely flowing out from the case 1a through the air inlet 2, especially in the case of the deodorizing apparatus shown in Fig. 7. Although the motor 11 may be operated continuously independently of the intermittent operation of the ozone generator 6, the motor 11 is driven intermittently as described above, so as to be serviceable for a prolonged period of time and also for savings in power.

With the deodorizing apparatus of Fig. 6, the residual ozone is converted to oxygen by the deodorizing catalyst 4, so that the motor 11 may be operated intermittently at the same interval as the ozone generator 6.

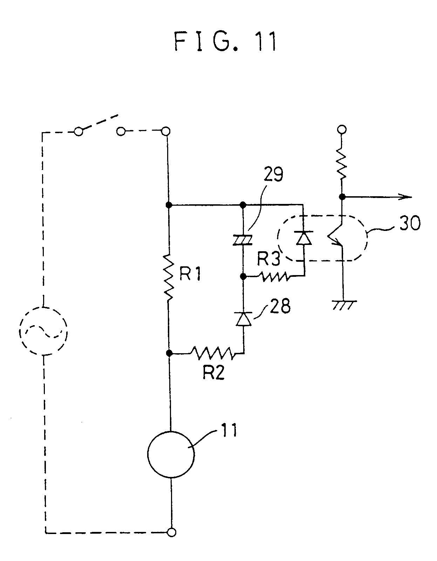

Next, with reference to Fig. 11, the current detecting circuit 26 comprises a resistor R1 connected in series with the motor 11, a series circuit connected in parallel with the resistor R1 and including a resistor R2, diode 28 and capacitor 29, and a series circuit connected to opposite ends of the capacitor 29 and including a resistor R3 and photocoupler 30. While the motor 11 is in normal operation, the resistor R1 produces a very low a.c. voltage, which is converted to a d.c. voltage by the diode 28 and the capacitor 29. Consequently, the light-emitting diode in the photocoupler 30 is turned on, and the phototransistor therein is also brought into conduction, thereby indicating to the microcomputer 24 that the motor 11 is in normal operation. If a break occurs in the motor 11, on the other hand, no current flows through the resistor R1, giving no voltage to the capacitor 29 and consequently turning off the light-emitting diode within the photocoupler 30. This indicates that the motor 11 is out of operation.

When a break in the motor 11 is detected, the high voltage generating unit 5 is deenergised, causing the ozone generator 6 to discontinue the production of ozone. More specifically, if a break occurs in the motor 11 which stops the fan 12 for one cause or another during the operation of the deodorizing apparatus, the current detecting circuit 26 detects this for the reason given above, whereupon the microcomputer 24 functions to deenergize the high voltage generating unit 5. Consequently, the ozone generator 6 produces no ozone, precluding the outflow of ozone due to the stopping of the motor 11.

The switching elements Sf and So are connected into the control circuit as will be described below. The switching element Sf is connected in series with a parallel circuit comprising the motor 11 and the series circuit of the high voltage generating unit 5 and the switching element So.

Accordingly, when an ON signal is intermittently emitted by the microcomputer 24, the switching elements Sf, So are closing to energize the high voltage generating unit 5 and the motor 11.

Suppose the switching element So is held turned on at all times owing to a malfunction. Even then, the high voltage generating unit 5 is not energised, and the ozone generator 6 produces no ozone unless the switching element Sf is turned on, i.e. unless the motor 11 is driven. Incidentally, should ozone be produced while the motor 11 is stopped, ozone would flow into the refrigerator and pose a great hazard especially in the case of the embodiments shown in Figs. 2 and 7.

Further if the switching element Sf malfunctions and does not close for one cause or another, the high voltage generating unit 5 similarly remains unenergised, and the ozone generator 6 produces no ozone.

The construction described above obviates the continuous production of ozone due, for example, to the malfunction or failure of the switching elements Sf, So, assuring the deodorizing apparatus of high safety.

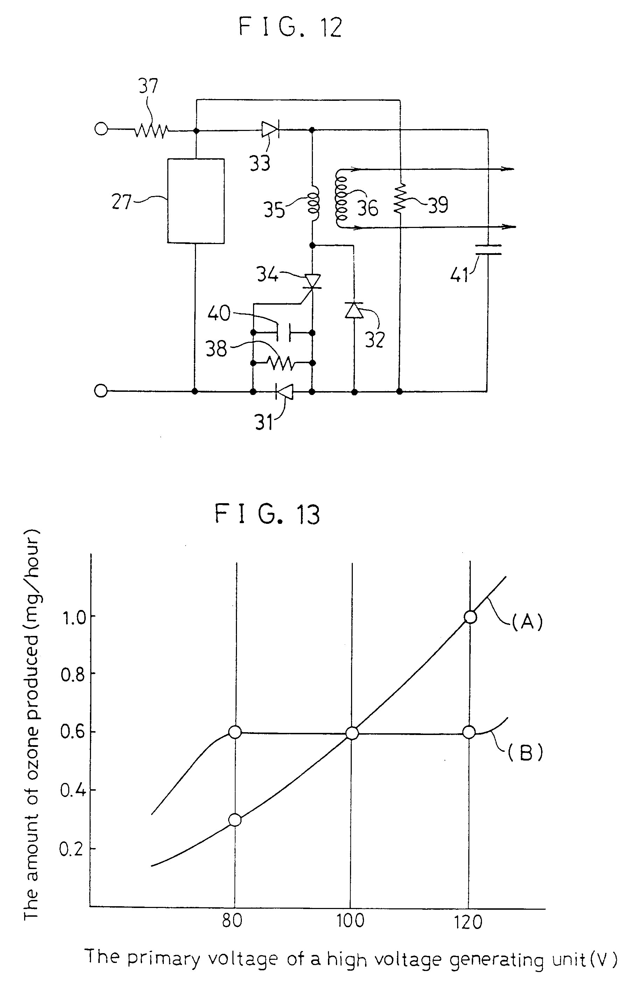

The high voltage generating unit 5 and the constant-voltage circuit 27 will be described next with reference to Figs. 12 and 13. Fig. 12 shows diodes 31, 32, 33, a thyristor 34 connected in series with a primary winding 35, a secondary winding 36 connected to the ozone generator 6, resistors 37, 38, 39 and capacitors 40, 41. A high voltage is applied to the ozone generator 6 by the charging and discharging of the capacitor 41 and the conduction and non-conduction of the thyristor 34.

The amount of ozone to be produced by the generator 6 varies with the voltage applied from the high voltage generating unit 5 and must therefore be made constant by applying a constant voltage to the generator 6. More specifically stated with reference to Fig. 13, curve (A), the amount of ozone to be produced varies with the primary voltage of the unit 5, which therefore needs to be made constant.

The constant-voltage circuit 27 provided for the high voltage generating unit 5 according to the invention makes it possible to apply a constant voltage to the ozone generator 6, enabling the generator to produce ozone in a constant amount as shown in Fig. 13, curve (B), and assuring the apparatus of a stabilised deodorizing operation.

The term "constant voltage" refers to the secondary voltage of the unit 5 whose variations are up to ±5% relative to a standard value even if the primary voltage of the unit varies in the range of 80 to 120 V.

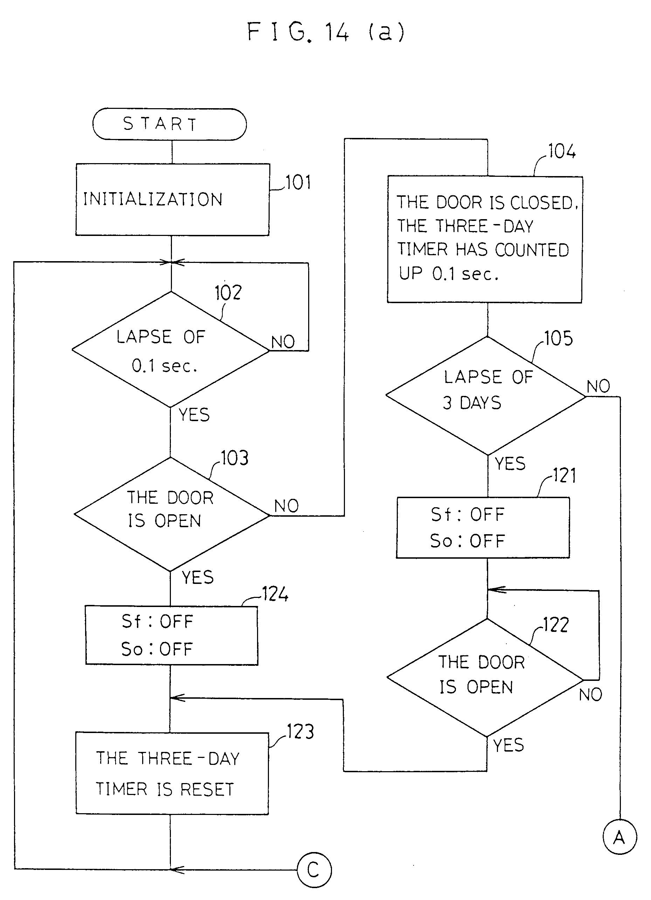

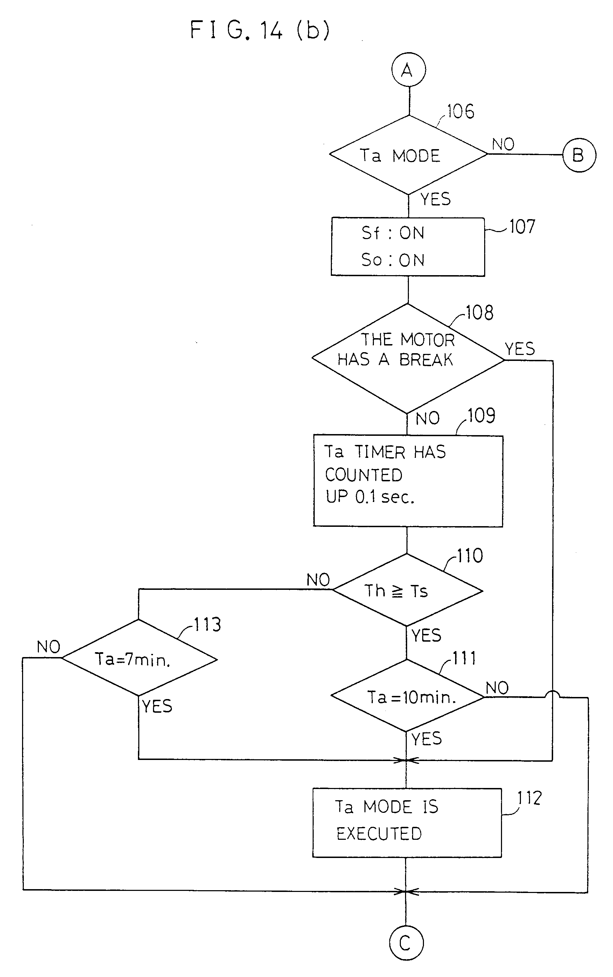

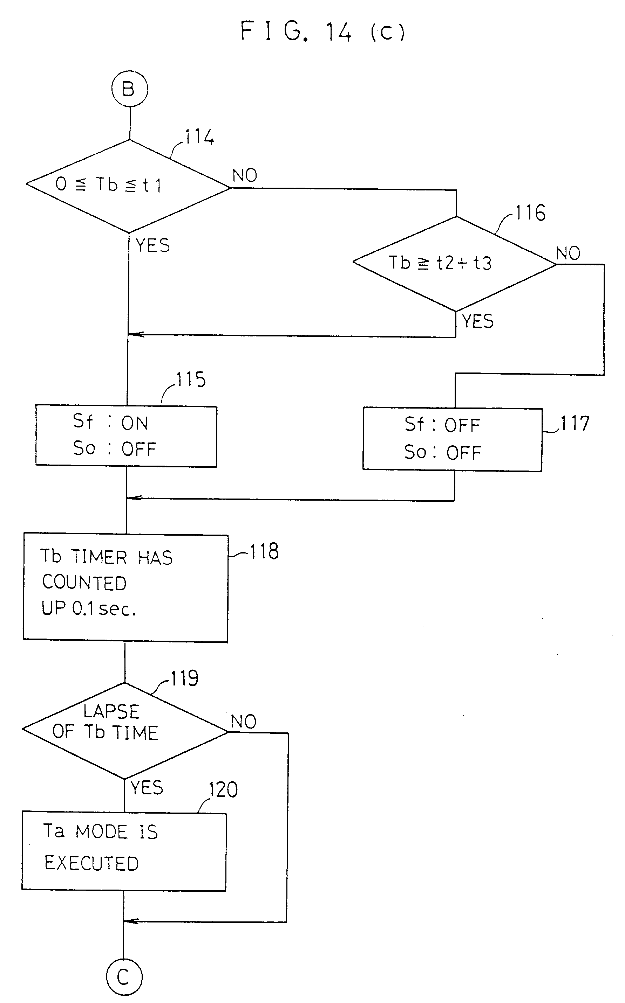

The operation of the deodorizing apparatus will be described in summary with reference to the flow charts of Fig. 14(a) to Fig. 14(c). When the apparatus is initiated into operation, the control system is initialised (step 101). The sequence shown in the flow charts proceeds from step to step at intervals of 0.1 second as measured by an internal timer of the microcomputer 24, so that upon lapse of 0.1 second (step 102), the door 22 is checked. If it is found closed with the door switch Sd off, the three-day timer is started (steps 103, 104). While the three-day timer is in counting operation (step 105), Ta mode follows (step 106), in which both the switching elements Sf, So are turned on (step 107). Unless the motor 11 has a break, the Ta timer is started (step 109). When the temperature Th, detected by the temperature sensor 23, is not lower than the set temperature Ts (5°C) (step 110) and upon lapse of 10 minutes, i.e. time Ta (step 111), Tb mode is executed (step 112). If Th is lower than Ts in step 110 and upon lapse of time Ta of 7 minutes (step 113), Tb mode is executed. In Tb mode, when the time Tb measured by the Tb timer is up to tl (step 114), the switching element Sf only is turned on. If $\text{tl < Tb < t2 + t3}$ , both the switching elements Sf, So are turned off, or if $\text{Tb ≧ t2 + t3}$ 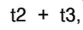 , the switching element Sf only is turned on (step 116 or 117). Upon the Tb timer counting up Tb time (20 minutes) (steps 118, 119), Ta mode follows again (step 120). When the door 22 has been held closed for three days (step 105), both the switching elements Sf, So are turned off (step 121). When the door 22 is subsequently opened (step 122), the three-day timer is reset (step 123). If the door 22 is opened in Ta or Tb mode (step 103), both the switching elements Sf, So are turned off, and the three-day timer is reset (steps 124, 123).

The deodorizing apparatus according to the present invention can be conveniently installed in refrigerators as explained above and also in other closed chambers such as cabins or automobiles.

The invention being thus described, it will be obvious that the same may be varied in many ways. Such variations are not to be regarded as a departure from the scope of the invention.

There are described above novel feature which the skilled man will appreciate give rise to advantages. These are each independent aspects of the invention to be covered by the present application, irrespective of whether or not they are included within the scope of the following claims.

## Claims

1. A deodorizing apparatus comprising means (11, 12) for generating an air flow through a case (1a) from an inlet (2) to an outlet (3) of the case (1a), means (6) for introducing ozone into the airflow at a position between said inlet (2) and outlet (3) and first means (10) disposed in said airflow downstream of said position for accelerating the decomposition of odours and ozone in the airflow, characterised by the provision of a second means (4) for accelerating the decomposition of odours and ozone located upstream of said position.

2. A deodorizing apparatus comprising means (11, 12) for generating an air flow through a case (1b) from an inlet (2) to an outlet (3) of the case (1b), means (6) for introducing ozone into the airflow at a position between said inlet (2) and outlet (3) and means (10) disposed in said airflow downstream of said position and for accelerating the decomposition of odours and ozone in the airflow, characterised in that said outlet (3) is above said inlet (2), and a motor (11) of said airflow generating means is located in the case (1b) upstream of said position.

3. An apparatus as defined in claim 1 wherein the air inlet (2), a motor (11), the second accelerating means (4), the ozone introducing means (6), the first accelerating means (10), a fan (12) and the air outlet (3) are arranged in this order in the air flow channel (1).

4. An apparatus as defined in claim 1 or claim 3 wherein a motor (11) of the air-flow generating means and the ozone introducing means are driven by the output voltage of a step-down transformer (14) which is connected to a commercial power supply.

5. An apparatus as defined in claim 4 wherein the step-down transformer (14) is housed in a case (13) separate from the case (1a) of the deodorizing apparatus.

6. An apparatus as defined in claim 5 wherein the case of the deodorizing apparatus is connected to the case (13) of the transformer (14) by a two-core cable (17).

7. An apparatus as defined in claim 6 wherein the two-core cable (17) is removably connected to the transformer case (13).

8. An apparatus as defined in any of claims 5 to 7 wherein the transformer case (13) has blades (15a, 15b) insertable into a commercial power supply receptacle for connecting the transformer (14) to the receptacle.

9. An apparatus as defined in claim 1 or claim 2 which is installed within a refrigerator having a door.

10. An apparatus as defined in claim 9 further comprising first control means for controlling the operation of a motor (11) of the air-flow generating means, and second control means for intermittently operating the ozone introducing means (6) with specified ON- and OFF-periods.

11. An apparatus as defined in claim 10 wherein the first control means intermittently operates the motor (11) in operative relation with the intermittent operation of the ozone introducing means (6) by the second control means.

12. An apparatus as defined in claim 10 wherein the first control means holds the motor (11) in operation for a period of time including and longer than the operation period of the ozone introducing means (6).

13. An apparatus as defined in any of claims 10 to 12 wherein the motor (11) is provided with current detecting means (26) included in its drive current circuit.

14. An apparatus as defined in claim 13 wherein the current detecting means (26) detects the absence of the drive current for the motor (11) and causes the second control means to stop the operation of the ozone introducing means (6).

15. An apparatus as defined in any of claims 1, 2 and 9 to 14 wherein the ozone introducing means (6) is provided with a high voltage generating circuit (5) having a constant-voltage circuit (27) at its power supply input side.

16. An apparatus as defined in any of claims 10 to 15 wherein a common a.c. power supply supplies drive power to the motor (11) and the ozone introducing means (6).

17. An apparatus as defined in claim 16 wherein the first control means has first switching means (Sf) for opening and closing a circuit for supplying the power from the a.c. power supply to the motor (11) and the ozone introducing means (6), and the second control means has second switching means (So) for opening and closing a circuit for supplying the power from the first switching means (Sf) to the ozone introducing means (6).

18. An apparatus as defined in any of claims 10 to 17 wherein the first and second control means include a microcomputer (24).

19. An apparatus as defined in any of claims 10 to 18 wherein the refrigerator has a temperature sensor (23) or a humidity sensor in its interior.

20. An apparatus as defined in claim 19 wherein the second control means is responsive to an output from the temperature sensor (23) or the humidity sensor to intermittently operate the ozone introducing means (6) with controlled ON- and OFF-periods.

21. An apparatus as defined in any of claims 10 to 20 wherein the refrigerator has a door switch (Sd) operative with the opening and closing of the door.

22. An apparatus as defined in claim 21 wherein the second control means has a first timer for measuring and setting the ON-period of the intermittent operation of the ozone introducing means (6), interrupts the measuring operation of the first timer and the intermittent operation of the ozone introducing means (6), and causes the first timer and the ozone introducing means (6) to resume their operations.

23. An apparatus as defined in claim 21 or claim 22 wherein the second control means has a second timer for measuring the period of time during which the door is closed, and stops the operation of the ozone introducing means (6) when the door is held closed for at least a predetermined period of time.

24. An apparatus as defined in any of claims 9 to 23 wherein the case (1a) is formed by a part of the inner wall of the refrigerator.

25. An apparatus as defined in claim 1 wherein the air inlet (2), the second accelerating means (4), the ozone introducing means (6), the first accelerating means (10), a motor (11), a fan (12) and the air outlet (3) are arranged in this order.

26. An apparatus as defined in claim 2 wherein the air flow channel is U-shaped, and the air inlet (2), a motor (11), the ozone introducing means (6) the accelerating means (10), a fan (12) and the air outlet (3) are arranged in this order.

27. An apparatus as defined in claim 2 wherein the air inlet (2), a motor (11), a fan (12), the ozone introducing means (6), the accelerating means (10) and the air outlet (3) are arranged in this order from the lower end of the apparatus upward against the direction of gravity.

28. An apparatus as defined in any preceding claim wherein the, or each, accelerating means (4, 10) is in the form of a plate having a honeycomb structure and is made of a material capable of accelerating the decomposition of odours and ozone.

29. A deodorizing apparatus comprising means (12) for generating an air flow through a case (1a) from an inlet (2) to an outlet (3) of the case (1a), means (6) for introducing ozone into the airflow at a position between said inlet (2) and outlet (3) and means (10) disposed in said airflow downstream of said position for accelerating the decomposition of odours and ozone in the airflow, characterised by control means (24) comprising temperature (23) or humidity detecting means, said control means (24) acting to control the amount of ozone introduced into said airflow in accordance with a detected temperature or humidity of the air to be deodorized.

## Patentansprüche

1. Desodoriervorrichtung mit Einrichtungen (11, 12) zur Erzeugung eines Luftstroms durch ein Gehäuse (1a) von einem Einlaß (2) zu einem Auslaß (3) des Gehäuses (1a), einer Einrichtung (6) zum Zugeben von Ozon in den Luftstrom an einer Stelle zwischen dem Einlaß (2) und dem Auslaß (3) und einer ersten Einrichtung (10), die in diesem Luftstrom stromab hinter dieser Stelle angeordnet ist, zur Beschleunigung des Abbaus der Gerüche und des Ozons in dem Luftstrom, **gekennzeichnet durch** eine zweite Einrichtung (4) zur Beschleunigung des Abbaus von Gerüchen und Ozon, die stromauf von der Stelle angeordnet ist.

2. Desodoriervorrichtung mit Einrichtungen (11, 12) zur Erzeugung eines Luftstroms durch ein Gehäuse (1b) von einem Einlaß (2) zu einem Auslaß (3) des Gehäuses (1b), einer Einrichtung (6) zum Zugeben von Ozon in den Luftstrom an einer Stelle zwischen dem Einlaß (2) und dem Auslaß (3) und einer Einrichtung (10), die in diesem Luftstrom stromab hinter dieser Stelle angeordnet ist, zur Beschleunigung des Abbaus der Gerüche und des Ozons in dem Luftstrom, **dadurch gekennzeichnet**, daß der Auslaß (3) oberhalb des Einlasses (2) angeordnet ist und ein zu den luftstromerzeugenden Einrichtungen gehörender Motor (11) in dem Gehäuse (1b) stromauf von der Stelle angeordnet ist.

3. Vorrichtung nach Anspruch 1, **dadurch gekennzeichnet**, daß der Lufteinlaß (2), ein Motor (11), die zweite Beschleunigungseinrichtung (4), die Ozon-zugebende Einrichtung (6), die erste Beschleunigungseinrichtung (10), ein Ventilator (12) und der Luftauslaß (3) in dieser Reihenfolge in dem Luftstromkanal (1) angeordnet sind.

4. Vorrichtung nach Anspruch 1 oder 3, **dadurch gekennzeichnet**, daß ein Motor (11) der luftstromerzeugenden Einrichtungen und die Ozon zugebende Einrichtung durch die Ausgangsspannung eines Abwärtstransformators (14), dervon einem üblichen Netzteil versorgt wird, angetrieben werden.

5. Vorrichtung nach Anspruch 4, **dadurch gekennzeichnet**, daß der Abwärtstransformator (14) in einem Gehäuse (13) getrennt von dem Gehäuse (1a) der Desodoriervorrichtung untergebracht ist.

6. Vorrichtung nach Anspruch 5, **dadurch gekennzeichnet**, daß das Gehäuse der Desodoriervorrichtung mit dem Gehäuse (13) des Transformators (14) durch ein zweiadriges Kabel (17) verbunden ist.

7. Vorrichtung nach Anspruch 6, **dadurch gekennzeichnet**, daß das zweiadrige Kabel (17) entfernbar mit dem Transformatorgehäuse (13) verbunden ist.

8. Vorrichtung nach einem der Ansprüche 5 bis 7, **dadurch gekennzeichnet**, daß das Transformatorgehäuse (13) Kontakte (15a, 15b) aufweist, die in eine übliche Steckdose einführbar sind, um den Transformator (14) mit der Steckdose zu verbinden.

9. Vorrichtung nach Anspruch 1 oder 2, **dadurch gekennzeichnet**, daß die Vorrichtung in einem Kühlschrank mit einer Tür untergebracht ist.

10. Vorrichtung nach Anspruch 9, **gekennzeichnet durch** eine erste Kontrolleinrichtung zur Steuerung des Betriebs eines Motors (11) der luftstromerzeugenden Einrichtungen, und einer zweiten Kontrolleinrichtung zum intermittierenden Betrieb der Ozon-zugebenden Einrichtung (6) mit bestimmten AN- und AUS-Perioden.

11. Vorrichtung nach Anspruch 10, **dadurch gekennzeichnet**, daß die erste Kontrolleinrichtung den Motor (11) intermittierend betreibt in Abhängigkeit vom intermittierenden Betrieb der Ozon-zugebenden Einrichtung (6) durch die zweite Kontrolleinrichtung.

12. Vorrichtung nach Anspruch 10, **dadurch gekennzeichnet**, daß die erste Kontrolleinrichtung den Motor (11) für einen Zeitraum in Betrieb hält, der den Betriebszeitraum der Ozon-zugebenden Einrichtung (6) umfaßt und länger ist als dieser.

13. Vorrichtung nach einem der Ansprüche 10 bis 12, **dadurch gekennzeichnet**, daß der Motor (11) eine stromanzeigende Einrichtung (26) aufweist, die in seinem Antriebsstromkreis angeordnet ist.

14. Vorrichtung nach Anspruch 13, **dadurch gekennzeichnet**, daß die stromanzeigende Einrichtung (26) das Fehlen des Antriebsstroms für den Motor (11) erkennt und die zweite Kontrolleinrichtung veranlaßt, den Betrieb der Ozon-zugebenden Einrichtung (6) zu stoppen.

15. Vorrichtung nach einem der Ansprüche 1, 2 und 9 bis 14, **dadurch gekennzeichnet**, daß die Ozon-zugebende Einrichtung (6) eine Hochspannungs-erzeugende Schaltung (5) aufweist, mit einer Konstantspannungsschaltung (27) an der Leistungseingangsseite.

16. Vorrichtung nach einem der Ansprüche 10 bis 15, **dadurch gekennzeichnet**, daß ein übliches Wechselspannungs-Netzteil den Motor (11) und die Ozon-zugebende Einrichtung (6) mit Antriebsenergie versorgt.

17. Vorrichtung nach Anspruch 16, **dadurch gekennzeichnet**, daß die erste Kontrolleinrichtung erste Schaltmittel (Sf) zum Öffnen und Schließen eines Schaltkreises zur Energieversorgung vom Wechselspannungs-Netzteil zum Motor (11) und zur Ozon-zugebenden Einrichtung (6) aufweist, und die zweite Kontrolleinrichtung zweite Schaltmittel (So) zum Öffnen und Schließen eines Schaltkreises zur Energieversorgung vom ersten Schaltmittel (Sf) zur Ozon-zugebenden Einrichtung (6) aufweist.

18. Vorrichtung nach einem der Ansprüche 10 bis 17, **dadurch gekennzeichnet**, daß die erste und die zweite Kontrolleinrichtung einen Mikrocomputer (24) aufweisen.

19. Vorrichtung nach einem der Ansprüche 10 bis 18, **dadurch gekennzeichnet**, daß das Kühlgerät einen Temperatursensor (23) oder einen Feuchtigkeitssensor in seinem Inneren aufweist.

20. Vorrichtung nach Anspruch 19, **dadurch gekennzeichnet**, daß die zweite Kontrolleinrichtung in Abhängigkeit von einer Messung des Temperatursensors (23) oder des Feuchtigkeitssensors die Ozon-zugebende Einrichtung (6) mit gesteuerten AN- und AUS-Perioden intermittierend betreibt.

21. Vorrichtung nach einem der Ansprüche 10 bis 20, **dadurch gekennzeichnet**, daß das Kühlgerät einen Türschalter (Sd) aufweist, der durch das Öffnen und Schließen der Tür betätigt wird.

22. Vorrichtung nach Anspruch 21, **dadurch gekennzeichnet**, daß die zweite Kontrolleinrichtung eine erste Schaltuhr zum Messen und Schalten der AN-Periode des intermittierenden Betriebs der Ozon-zugebenden Einrichtung (6) aufweist, den Messbetrieb der ersten Schaltuhr und den intermittierenden Betrieb der Ozon-zugebenden Einrichtung (6) unterbricht, und die die erste Schaltuhr und die Ozon-zugebende Einrichtung (6) veranlaßt, ihren Betrieb wiederaufzunehmen.

23. Vorrichtung nach Anspruch 21 oder 22, **dadurch gekennzeichnet**, daß die zweite Kontrolleinrichtung eine zweite Schaltuhr zum Messen des Zeitabschnitts, in dem die Tür geschlossen ist, aufweist, und die den Betrieb der Ozon-zugebenden Einrichtung (6) stoppt, wenn die Tür für mindestens eine bestimmte Zeitdauer geschlossen bleibt.

24. Vorrichtung nach einem der Ansprüche 9 bis 23, **dadurch gekennzeichnet**, daß das Gehäuse (1a) durch einen Teil der Innenwand des Kühlgeräts gebildet wird.

25. Vorrichtung nach Anspruch 1, **dadurch gekennzeichnet**, daß der Lufteinlaß (2), die zweite Beschleunigungseinrichtung (4), die Ozon-zugebende Einrichtung (6), die erste Beschleunigungseinrichtung (10), ein Motor (11), ein Ventilator (12) und ein Luftauslaß (3) in dieser Reihenfolge angeordnet sind.

26. Vorrichtung nach Anspruch 2, **dadurch gekennzeichnet**, daß der Luftstromkanal U-förmig ist und daß der Lufteinlaß (2), ein Motor (11), die Ozon-zugebende Einrichtung (6), die Beschleunigungseinrichtung 10), ein Ventilator (12) und der Luftauslaß (3) in dieser Reihenfolge angeordnet sind.

27. Vorrichtung nach Anspruch 2, **dadurch gekennzeichnet**, daß der Lufteinlaß (2), ein Motor (11), ein Ventilator (12), die Ozon-zugebende Einrichtung (6), die Beschleunigungseinrichtung (10) und der Luftauslaß (3) in dieser Reihenfolge vom unteren Ende der Vorrichtung aufwärts, gegen die Schwerkraftrichtung angeordnet sind.

28. Vorrichtung nach einem der vorstehenden Ansprüche, **dadurch gekennzeichnet**, daß eine oder jede der Beschleunigungseinrichtungen (4, 10) die Form einer Platte mit einer Bienenwabenstruktur aufweist und aus einem Material zur Beschleunigung der Trennung von Gerüchen und Ozon hergestellt ist.

29. Desodoriervorrichtung mit einer Einrichtung (12) zur Erzeugung eines Luftstroms durch ein Gehäuse (1a) von einem Einlaß (2) zu einem Auslaß (3) des Gehäuses (1a), einer Einrichtung (6) zum Zugeben von Ozon in den Luftstrom an einer Stelle zwischen dem Einlaß (2) und dem Auslaß (3) und einer Einrichtung (10), die in diesem Luftstrom stromabwärts hinter dieser Stelle angeordnet ist, zur Beschleunigung der Trennung der Gerüche und des Ozons in dem Luftstrom, **gekennzeichnet durch** eine Steuerungseinrichtung (24), die eine Einrichtung zum Erfassen von Temperatur (23) oder Feuchtigkeit aufweist und die die Menge von Ozon, das dem Luftstrom zugegeben wird, in Abhängigkeit von einer erfaßten Temperatur oder Feuchtigkeit der zu desodorierenden Luft regelt.

## Revendications

1. Appareil désodorisant, comprenant des moyens (11, 12) pour produire un écoulement d'air à travers un boîtier (1a), depuis une entrée (2) vers une sortie (3) du boîtier (1a), un moyen (6) pour introduire de l'ozone dans l'écoulement d'air, en une position située entre lesdites entrée (2) et sortie (3), et un premier moyen (10), disposé dans ledit écoulement d'air, en aval de ladite position, pour accélérer la décomposition d'odeurs et de l'ozone dans l'écoulement d'air, caractérisé par l'agencement d'un second moyen (4) pour accélérer la décomposition d'odeurs et de l'ozone, situé en aval de ladite position.

2. Appareil désodorisant comprenant des moyens (11,12) pour produire un écoulement d'air à travers un boitier (1b), depuis une entrée (2) vers une sortie (3) du boîtier (1b), un moyen (6) pour introduire de l'ozone dans l'écoulement d'air, en une position située entre lesdites entrée (2) et sortie (3) et un moyen (10) disposé dans ledit écoulement d'air, en aval de ladite position et pour accélérer la décomposition d'odeurs et de l'ozone dans l'écoulement d'air, caractérisé en ce que ladite sortie (3) est située au-dessus de ladite entrée (2), et un moteur (11) dudit moyen de production d'écoulement d'air est situé dans le boîtier (1b), en amont de ladite position.

3. Appareil selon la revendication 1, dans lequel l'entrée d'air (2), un moteur (11), le second moyen d'accélération (4), le moyen d'introduction d'ozone (6), le premier moyen d'accélération (10), un ventilateur (12), et la sortie d'air (3) sont disposés dans cet ordre, dans le canal d'écoulement d'air (1).

4. Appareil selon la revendication 1 ou 3, dans lequel un moteur (11) du moyen de production d'écoulement d'air et le moyen d'introduction d'ozone sont entraînés par la tension de sortie d'un transformateur dévolteur (14), qui est relié à une source d'alimentation du commerce.

5. Appareil selon la revendication 4, dans lequel le transformateur dévolteur (14) est logé dans un boîtier (13), séparé du boîtier (1a) de l'appareil désodorisant.

6. Appareil selon la revendication 5, dans lequel le boîtier de l'appareil désodorisant est relié au boîtier (13) du transformateur (14), par un câble à deux conducteurs (17)

7. Appareil selon la revendication 6, dans lequel le câble à deux conducteurs (17) est relié de façon amovible au boîtier de transformateur (13).

8. Appareil selon l'une quelconque des revendications 5 à 7, dans lequel le boîtier de transformateur (13) présente des lames (15a, 15b) pouvant être insérées dans une fiche femelle de source d'alimentation du commerce, pour relier le transformateur (14) à la fiche femelle.

9. Appareil selon la revendication 1 ou 2, installé dans un réfrigérateur présentant une porte.

10. Appareil selon la revendication 9, comprenant en outre un premier moyen de commande, pour commander le fonctionnement d'un moteur (11) du moyen de production d'écoulement d'air, et un second moyen de commande pour actionner de façon intermittente le moyen d'introduction d'ozone (6), avec des périodes MARCHE et ARRET spécifiées.

11. Appareil selon la revendication 10, dans lequel le premier moyen de commande actionne de façon intermittente le moteur (11), en relation fonctionnelle avec le fonctionnement intermittent du moyen d'introduction d'ozone (6) à l'aide du second moyen de commande.

12. Appareil selon la revendication 10, dans lequel le premier moyen de commande maintient le moteur (11) en fonctionnement, pendant une période de temps comprenant, et plus longue que, la période de fonctionnement du moyen d'introduction d'ozone (6).

13. Appareil selon l'une quelconque des revendications 10 à 12, dans lequel le moteur (11) est pourvu d'un moyen de détection de courant (26), inclus dans son circuit de courant d'entraînement.

14. Appareil selon la revendication 13, dans lequel le moyen de détection de courant (26) détecte l'absence du courant d'entraînement pour le moteur (11) et provoque l'arrêt du fonctionnement du moyen d'introduction d'ozone (6), par le second moyen de commande.

15. Appareil selon l'une quelconque des revendications 1, 2, et 9 à 14, dans lequel le moyen d'introduction d'ozone (6) est pourvu d'un circuit de production de haute tension (5), présentant un circuit à tension constante (27) sur son côté d'entrée de source d'alimentation.

16. Appareil selon l'une quelconque des revendications 10 à 15, dans lequel une source d'alimentation de courant alternatif commune fournit une puissance d'entraînement au moteur (11) et au moyen d'introduction d'ozone (6).

17. Appareil selon la revendication 16, dans lequel le premier moyen de commande présente un premier moyen de commutation (SF), pour ouvrir et fermer un circuit servant à fournir au moteur (11) et au moyen d'introduction d'ozone (6) de la puissance provenant de la source d'alimentation à courant alternatif, et le second moyen de commande présente un seconde moyen de commutation (SO), pour ouvrir et fermer un circuit servant à fournir de la puissance, du premier moyen de commutation (SF) au moyen d'introduction d'ozone (6).

18. Appareil selon l'une quelconque des revendications 10 à 17, dans lequel les premier et second moyens de commande comprennent un micro-ordinateur (24).

19. Appareil selon l'une quelconques revendications 10 à 18, dans lequel le réfrigérateur présente en son sein un capteur de température (23) ou un capteur d'humidité.

20. Appareil selon la revendication 19, dans lequel le second moyen de commande est sensible à un signal de sortie du capteur de température (23) ou du capteur d'humidité, pour actionner par intermittence le moyen d'introduction d'ozone (6) selon des périodes MARCHE et ARRET commandées.

21. Appareil selon l'une quelconque des revendications 10 à 20, dans lequel le réfrigérateur présente un interrupteur de porte (Sd), fonctionnant à l'ouverture et à la fermeture de la porte.

22. Appareil selon la revendication 21, dans lequel le second moyen de commande présente une première minuterie pour mesurer et établir la période MARCHE du fonctionnement intermittent du moyen d'introduction d'ozone (6), interrompre l'opération de comptage de la première minuterie et le fonctionnement intermittent du moyen d'introduction d'ozone (6) et provoquer le rétablissement des fonctionnements de la première minuterie et du moyen d'introduction d'ozone (6).

23. Appareil selon la revendication 21 ou 22, dans lequel le second moyen de commande présente une deuxième minuterie pour mesurer la période de temps durant laquelle la porte est fermée et stopper le fonctionnement du moyen d'introduction d'ozone (6) lorsque la porte est maintenue fermée pendant au moins une période de temps prédéterminée.

24. Appareil selon l'une quelconques des revendications 9 à 23, dans lequel le boîtier (1a) est constitué d'une partie de la paroi intérieure du réfrigérateur.

25. Appareil selon la revendication 1, dans lequel l'entrée d'air (2), le second moyen d'accélération (4), le moyen d'introduction d'ozone (6), le premier moyen d'accélération (10), un moteur (11), un ventilateur (12) et la sortie d'air sont disposés dans cet ordre.

26. Appareil selon la revendication 2, dans lequel le canal d'écoulement d'air est en forme de U et l'entrée d'air (2), un moteur (11), le moyen d'introduction d'ozone (6), le moyen d'accélération (10), un ventilateur (12) et la sortie d'air (13) sont disposés dans cet ordre.

27. Appareil selon la revendication 2, dans lequel l'entrée d'air (2), un moteur (11), un ventilateur (12), le moyen d'introduction d'ozone (6), le moyen d'accélération (10) et la sortie d'air (13) sont disposés dans cet ordre, depuis l'extrémité inférieure de l'appareil, vers le haut, à l'opposé du sens de la gravité.

28. Appareil selon l'une quelconque des revendications précédentes, dans lequel le ou chaque moyen d'accélération (4, 10) se présente sous la forme d'une plaque présentant une structure d'alvéoles et est réalisée en un matériau capable d'accélérer la décomposition d'odeurs et de l'ozone.

29. Appareil désodorisant, comprenant un moyen (12) pour produire un écoulement d'air à travers un boîtier (1a), depuis une entrée (2) à une sortie (3) du boîtier (1a), un moyen (6) pour introduire de l'ozone dans l'écoulement d'air, en une position située entre lesdites entrée (2) et sortie (3), et un moyen (10) disposé dans ledit écoulement d'air en aval de ladite position, pour accélérer la décomposition d'odeurs et de l'ozone dans l'écoulement, caractérisé par un moyen de commande (24) comprenant un moyen de détection de température (23) ou d'humidité, ledit moyen de commande (24) fonctionnant pour commander la quantité d'ozone introduite dans ledit écoulement d'air, en fonction d'une température ou humidité mesurée de l'air à désodoriser.
